# EUROPEAN PATENT APPLICATION

(11) **EP 4 173 622 A1**
(43) Date of publication of application: **03.05.2023**
(21) Application number: 21854486.4
(22) Date of filing: 04.08.2021
(51) Int. Cl.: A61K 31/4725, A61K 31/197, A61K 9/00, A61P 19/02, A61P 29/00

(54) **USE OF CASPASE INHIBITOR FOR ALLEVIATING OR TREATING OSTEOARTHRITIS**

(30) Priority: 05.08.2020 KR 20200098151
(71) Applicant: Lg Chem, Ltd., Seoul 07336 (KR)
(72) Inventor: BAEK, Jae Uk, Seoul 07796 (KR); PARK, Jung Gyu, Daejeon 34013 (KR); KIM, Sung Won, Seoul 07796 (KR); PARK, Hyun Seo, Seoul 07796 (KR); CHOI, Sei Hyun, Daejeon 34013 (KR); JIN, Myung Won, Seoul 07796 (KR); LEE, Min Kyoung, Seoul 07796 (KR)
(74) Representative: Hoffmann Eitle
(86) International application number: PCT/KR2021/010209
(87) International publication number: WO 2022/030987

(57) **Abstract**

The present invention relates to a use of a caspase inhibitor for alleviating or treating osteoarthritis.

## Description

### TECHNICAL FIELD

The present invention relates to the use of a caspase inhibitor in alleviating or treating osteoarthritis.

### BACKGROUND ART

Caspases are cysteine proteases that exist in the form of an α2β2 tetramer. Caspase-1 (ICE), one of them, is a kind of cytokines and participates in converting inactive prointerleukin-1β to active interleukin-1β. Interleukin-1 includes interleukin-1α and interleukin-1β, both of which are synthesized in monocytes in the form of a precursor having 31kDa. Only prointerleukin-1β is activated by ICE. The positions hydrolyzed by caspase-1 are Asp²⁷-Gly²⁸ and Asp¹¹⁶-Ala¹¹⁷. The hydrolysis of the latter position gives interleukin-1β. Interleukin-1β has been reported to act as an important mediator in causing inflammation. Caspase-1 was discovered for the first time in 1989, and in two independent study groups, the three-dimensional structure thereof was determined by X-ray crystallographic method.

Caspase-3 (CPP-32) is broadly studied for its role or mechanism for action, and its three-dimensional structure was determined in 1996. Caspase-3 (apopain) activated from procaspase-3 hydrolyzes (P4)Asp-X-X-Asp(P1) motif, and the known substrates include poly(ADP-ribose) polymerase, U1 70,000 Mr small nuclear ribonucleoprotein and catalytic subunit of 460,000 Mr DNA-dependent protein kinase, etc. The X-ray structure of caspase-7 has been reported to be very similar to that of caspase-3.

Caspase-8 and 9 are present in the upstream of caspase-3, 6 and 7, and these caspases are known to participate in the apoptosis cascade. The X-ray structure of caspase-8 was determined in 1999, and particularly the inhibitors thereof may be advantageously used for treating diseases related to apoptosis.

Caspase inhibitors refer those compounds that inhibit the activity of caspase, and so control such symptoms as inflammation, apoptosis, etc. caused by caspase activity. Among caspase inhibitors, an irreversible inhibitor is known to show more effective inhibition activity since it irreversibly inactivates an enzyme to control apoptosis. As diseases associated with caspases, dementia, stroke, diabetes, gastric ulcer, ischemic heart disease, etc. have been known.

Osteoarthritis is a disease caused by breakdown of joint cartilage and underlying bone tissue, and common symptoms are joint pain and stiffness. Initially, pain is felt only when moving, but as it becomes chronic, pain is felt continuously. Up to now, there is no fundamental treatment for osteoarthritis, and as symptomatic therapy, intra-articular steroids for pain relief, oral or topical non-steroid anti-inflammatory drugs (NSAIDs) and the like have been used. However, in the case of steroids, their use is limited due to various side effects that affect body metabolism, and if used repeatedly, resistance to the effect gradually develops. Recently, a sustained-release formulation of steroid has been approved, but the safety in long-term use is being carefully monitored. In addition, in the case of oral NSAIDs, their use is limited due to gastrointestinal adverse effects and increased cardiovascular risk.

### DISCLOSURE OF INVENTION

### TECHNICAL PROBLEM

Accordingly, the technical problem of the present invention is the provision of a composition for alleviating or treating osteoarthritis-for which there is no fundamental treatment and only symptomatic treatment for pain relief-which can show long-term analgesic effect and improvement of cartilage function and structure with only a single administration without the risk of side effects or resistance to the effects of conventionally used therapeutic agents.

### SOLUTION TO PROBLEM

To solve the above technical problem, the present invention provides a pharmaceutical composition for alleviating or treating osteoarthritis having long-lasting effect with a single administration, which comprises a therapeutically effective amount of a caspase inhibitor along with a pharmaceutically acceptable carrier.

In addition, the present invention provides a method for alleviating or treating osteoarthritis for a long time, comprising administering a therapeutically effective amount of a caspase inhibitor to a subject in need thereof with a single administration.

Furthermore, the present invention provides use of a caspase inhibitor in alleviating or treating osteoarthritis for a long time with a single administration.

Hereinafter, the present invention is described in detail.

The present invention provides a pharmaceutical composition for alleviating or treating osteoarthritis having long-lasting effect with a single administration, which comprises a therapeutically effective amount of a caspase inhibitor along with a pharmaceutically acceptable carrier.

Caspase inhibitors have attracted attention as hepatocyte-protective drugs or therapeutic agents for inflammation-related diseases by interfering with the activity of caspases, which plays an essential role in apoptosis, necrosis and inflammation. The present inventors have accomplished the present invention by confirming the surprising result that when a caspase inhibitor is applied to osteoarthritis, it can show not only the long-term analgesic effect but also the effect of improving the cartilage structure with only a single administration.

According to one embodiment of the present invention, the caspase inhibitor is selected from the group consisting of nivocasan, IDN-1965, emricasan, MX-1013, pralnacasan, RU-36384, belnacasan and a pharmaceutically acceptable salt thereof, but is not limited thereto.

Nivocasan is (R)-N-((2S,3S)-2-(fluoromethyl)-2-hydroxy-5-oxotetrahydrofuran-3-yl]-5-isopropyl-3-(isoquinolin-1-yl)-4,5-dihydroisoxazole-5-carboxamide of the following Formula 1.

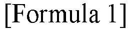

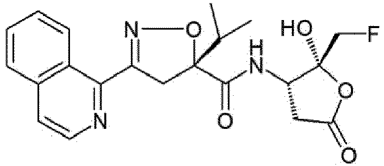

IDN-1965 is 3-[2(S)-(1,3-dimethyl-1H-indol-2-ylcarboxamido)-3-methylbutanamido]-5-fluoro-4-oxopentanoic acid of the following Formula 2.

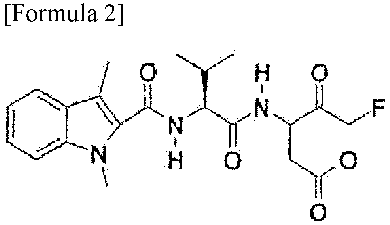

Emricasan (IDN-6556) is N-(2-tert-butylphenyl)-2-oxoglycyl-N-[1(S)-(carboxymethyl)-2-oxo-3-(2,3,5,6-tetrafluorophenoxy)propyl]-L-alaninamide of the following Formula 3.

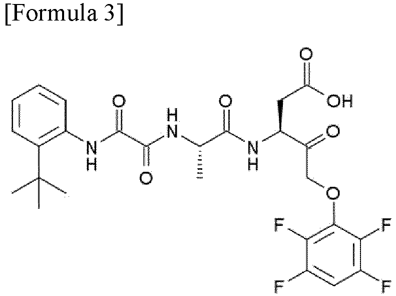

MX-1013 is N-(benzyloxycarbonyl)-L-valyl-DL-aspartylfluoromethane of the following Formula 4.

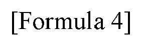

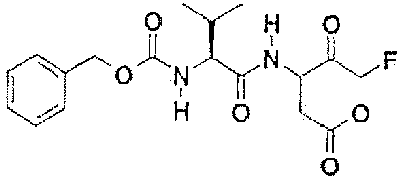

Pralnacasan is N-[2(R)-Ethoxy-5-oxotetrahydrofuran-3(S)-yl]-9(S)-(1-isoquinolinylcarboxamido)-6,10-dioxooctahydro-6H-pyridazino[1,2-a][1,2]diazepine-1(S)-carboxamide of the following Formula 5.

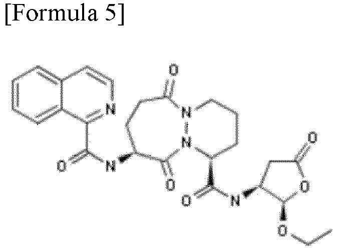

RU-36384 is 3(S)-[9(S)-(1-isoquinolinylcarboxamido)-6,10-dioxooctahydro-6H-pyridazino[1,2-a][1,2]diazepin-1(S)-ylcarboxamido]-4-oxobutanoic acid of the following Formula 6.

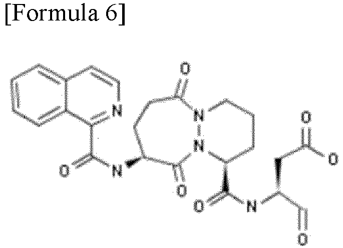

Belnacasan is N-(4-amino-3-chlorobenzoyl)-3-methyl-L-valyl-N-[2(R)-ethoxy-5-oxoperhydrofuran-3(S)-yl]-L-prolinamide of the following Formula 7.

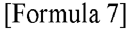

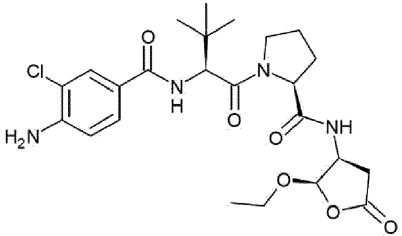

According to another embodiment of the present invention, the caspase inhibitor is nivocasan or a pharmaceutically acceptable salt thereof. The method for preparing nivocasan is described in detail in International Publication No. WO 2006/090997 A1 (publication date: August 31, 2006), which is incorporated herein by reference.

In another embodiment according to the present invention, examples of the pharmaceutically acceptable salt of nivocasan include, but are not limited to, inorganic acids such as hydrochloric acid, sulfuric acid, nitric acid, phosphoric acid, hydrobromic acid, hydroiodic acid; organic carbonic acids such as tartaric acid, formic acid, citric acid, acetic acid, trichloroacetic acid, trifluoroacetic acid, gluconic acid, benzoic acid, lactic acid, fumaric acid, maleic acid; acid addition salts formed by sulfonic acids such as methanesulfonic acid, benzenesulfonic acid, p-toluenesulfonic acid or naphthalenesulfonic acid.

According to another embodiment of the present invention, the caspase inhibitor is emricasan or a pharmaceutically acceptable salt thereof.

In another embodiment according to the present invention, the long-lasting effect is analgesic or pain-reducing effect.

In another embodiment according to the present invention, the long-lasting effect is cartilage structure improvement effect.

The compounds according to the present invention can be formulated as various pharmaceutically administered dosage forms. In the preparation of the pharmaceutical composition of the present invention, an active ingredient-specifically, a caspase inhibitor or a pharmaceutically acceptable salt thereof-is mixed with selected pharmaceutically acceptable carriers considering the dosage form to be prepared. For example, the pharmaceutical composition of the present invention can be formulated as injections, oral preparations and the like, as needed.

In another embodiment according to the present invention, the pharmaceutical composition is an injection formulation. In the present invention, injection formulations-for example, sterilized aqueous- or oil-based suspension for injection-may be prepared according to known techniques by using an appropriate dispersing agent, wetting agent or suspending agent. The solvents useful for this purpose include water, ringer solution and isotonic NaCl solution, and sterilized, immobilized oils are also used as a solvent or a suspending medium conventionally. Any non-irritant immobilized oils including mono- and di-glycerides may be used for this purpose, and fatty acids such as an oleic acid or polysorbate 80 (Tween 80) may be used for an injection formulation.

In another embodiment according to the present invention, the injection formulation is for topical administration

In another embodiment according to the present invention, the injection formulation is administered into the joint cavity.

In another embodiment according to the present invention, the administration effect of the pharmaceutical composition may last for a long time-for example, 1 month or more, 2 months or more, 3 months or more, 4 months or more, 5 months or more, or 6 months or more.

In another embodiment according to the present invention, the pharmaceutical composition may be administered with an interval of 4 weeks or more, 5 weeks or more, 6 weeks or more, 7 weeks or more, 8 weeks or more, 9 weeks or more, 10 weeks or more, 11 weeks or more, 12 weeks or more, 13 weeks or more, 14 weeks or more, 15 weeks or more, 16 weeks or more, 17 weeks or more, 18 weeks or more, 19 weeks or more, 20 weeks or more, 21 weeks or more, 22 weeks or more, 23 weeks or more, 24 weeks or more, or 25 weeks or more.

In another embodiment according to the present invention, the pharmaceutical composition may be prepared for administration to a mammal. In another embodiment according to the present invention, the mammal is a human.

In another embodiment according to the present invention, a "therapeutically effective amount" for an individual subject refers to an amount sufficient to achieve the above pharmacological effect-i.e., the therapeutic effect as described above. The amount of the compound may vary depending on the condition and severity of the subject, the mode of administration, and the age of the subject to be treated, but could be determined by persons of ordinary skill in the art based on their knowledge.

In another embodiment according to the present invention, the nivocasan may be administered into a human in a dose of 1 to 50 mg, based on a free base form. In another embodiment according to the present invention, the nivocasan may be administered into a human in a dose of 1 mg or more, 2 mg or more, 3 mg or more, 4 mg or more, 5 mg or more, 6 mg or more, 7 mg or more, 8 mg or more, 9 mg or more, 10 mg or more, 11 mg or more, 12 mg or more, 13 mg or more, 14 mg or more, 15 mg or more, 16 mg or more, 17 mg or more, 18 mg or more, 19 mg or more, 20 mg or more, 30 mg or less, 31 mg or less, 32 mg or less, 33 mg or less, 34 mg or less, 35 mg or less, 36 mg or less, 37 mg or less, 38 mg or less, 39 mg or less, 40 mg or less, 41 mg or less, 42 mg or less, 43 mg or less, 44 mg or less, 45 mg or less, 46 mg or less, 47 mg or less, 48 mg or less, 49 mg or less, 50 mg or less, or a combination thereof, based on a free base form.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body.

Herein, a "carrier" means a compound that facilitates the addition of compounds into the cell or tissue. For example, dimethylsulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

Herein, the term "alleviation" refers to ameliorating a disease and/or its accompanying symptoms altogether or in part.

Herein, the term "treatment" is used to mean deterring, delaying or ameliorating the progress of diseases in a subject exhibiting symptoms of diseases.

### EFFECTS OF THE INVENTION

The pharmaceutical composition according to the present invention can effectively alleviate or treat osteoarthritis by having no risk of side effects and showing an effect of improving the function and structure of cartilage as well as an analgesic effect for a long period of time with only a single administration without resistance to the effect. Specifically, in the case of an injection formulation with high invasiveness and great pain or discomfort felt by a subject, the present pharmaceutical composition-in which a single administration can show long-term osteoarthritis relief or therapeutic efficacy, so the administration interval can be long such as 12 weeks or more-can reduce pain of patients and save costs.

### BRIEF DESCRIPTION OF DRAWINGS

Figure 1 is a graph showing the results of measuring the suppressive efficacy against IL-1β secretion induced by LPS/nigericin in THP-1 cells.
Figure 2 is a graph showing the results of measuring the suppressive efficacy against IL-1β secretion induced by LPS in human-derived peripheral blood mononuclear cells.
Figure 3 is a graph showing the results of measuring the preventive effect on apoptosis by TNF-α in human-derived chondrocytes.
Figure 4 is a graph showing the results of measuring the efficacy of improving walking ability after intra-articular administration of nivocasan in an animal model of osteoarthritis.
Figure 5 is a graph showing the results of measuring the efficacy of improving walking ability after a single intra-articular administration of emricasan in an animal model of osteoarthritis compared with nivocasan.
Figure 6 is a graph showing the results of evaluating the effect of nivocasan and emricasan on the joint structure after a single intra-articular administration in an animal model of osteoarthritis by MRI.
Figure 7 is a graph showing the results of histopathological evaluation of the effects on the joint after autopsy in an animal model of osteoarthritis.

### MODE FOR THE INVENTION

Hereinafter, the present invention is explained in more detail with the following examples. However, it must be understood that the protection scope of the present invention is not limited to the examples.

### Example 1: Suppressive efficacy against LPS/nigericin-induced IL-1β secretion in THP-1 cells

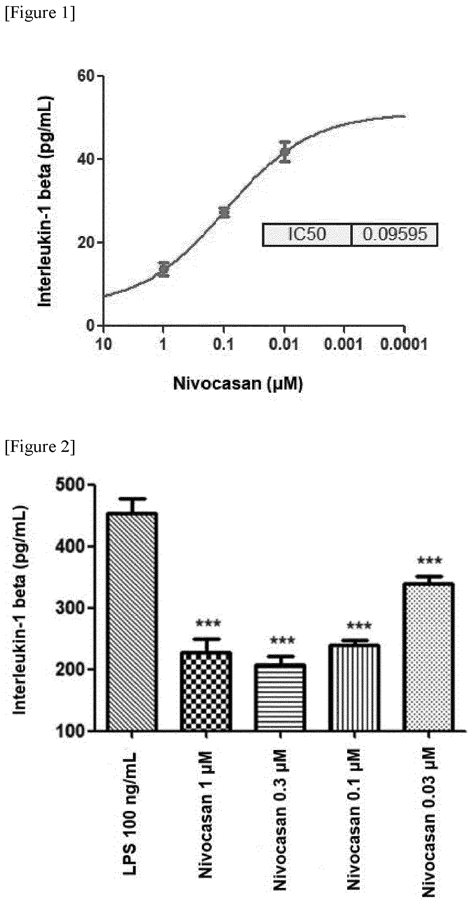

To evaluate the inflammation blocking effects of nivocasan, IL-1β secretion was measured in THP-1 cells under conditions in which inflammation was induced by lipopolysaccharide (LPS)/nigericin treatment. The day before LPS treatment, THP-1 cells were plated at 2.5×10⁵ cells/well (24-well plate) in RPMI-1640 media and treated with 0.5 µM phorbol 12-myristate 13-acetate (PMA) for macrophage differentiation during overnight culture. The media was then changed with Opti-MEM media and the cells were incubated with LPS 100 ng/ml (the first inflammation signal for pro-IL-1β up-regulation) for 24 hours. The cells were then washed with PBS twice and treated with nivocasan or DMSO for 30 minutes in Opti-MEM media, followed by incubation with 5 µM nigericin & 1 mM ATP for 1 hour to activate inflammasome (NLRP3 activation and IL-1β secretion). Secreted IL-1β was measured from cell media by using R&D Systems Quantikine human IL-1β (detection limit: 7.8 pg/ml), and the results are represented in Figure 1. As can be seen from Figure 1, it was confirmed that nivocasan suppressed LPS/nigericin-induced IL-1β secretion in THP-1 cells in a dose-dependent manner.

### Example 2: Suppressive efficacy against LPS-induced IL-1β secretion in human-derived PBMC

The inhibitory effects of nivocasan on inflammasome activation in human peripheral blood mononuclear cells (PBMCs) were evaluated by measuring LPS-induced IL-1β secretion. Freshly isolated PBMCs were plated at 2.5×10⁵ cells/well (24-well plate) in RPMI-1640 media and treated with nivocasan or DMSO for 30 minutes, followed by incubation with 100 ng/ml LPS for 24 hours to induce inflammation. Levels of IL-1β in PBMC supernatants was measured by ELISA (R&D Systems Quantikine human IL-1β; detection limit: 7.8 pg/ml), and the results are represented in Figure 2. As can be seen from Figure 2, it was confirmed that nivocasan suppressed LPS-induced IL-1β secretion from PBMCs in a dose dependent manner, showing maximum effect at 0.3 µM.

### Example 3: Protective effect on TNF-α-induced apoptosis in human-derived chondrocytes

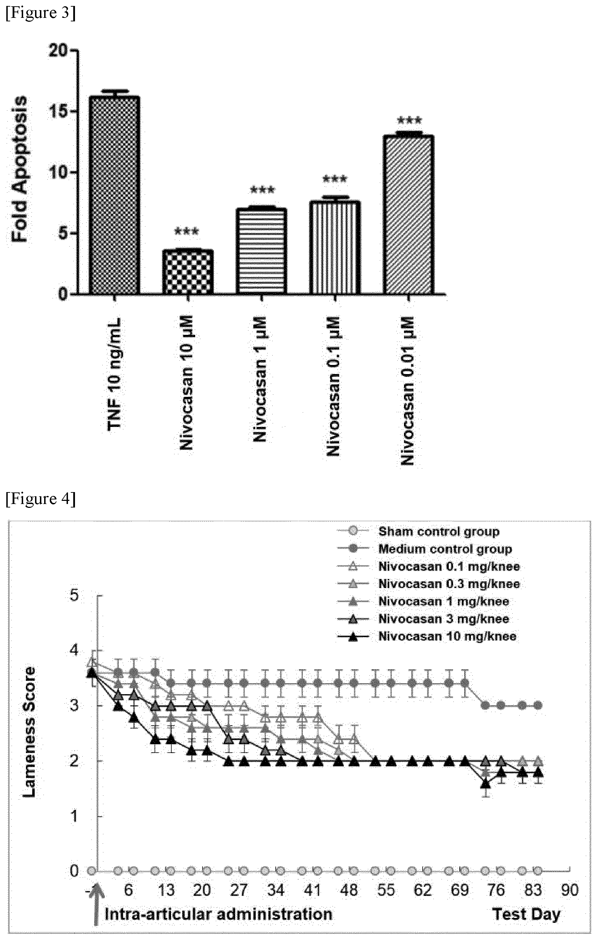

The protective effect of nivocasan on TNF-α-induced apoptosis was evaluated in human chondrocytes. Human normal chondrocytes were plated at 1×10⁵ cells/well (collagen-coated 24-well plate) in chondrocyte media (Prigrow IV Medium, Applied Biological Materials Inc.) and incubated overnight. The cells were treated with nivocasan or DMSO for 30 minutes, followed by incubation with 10 ng/ml human recombinant TNF-α and 1 µg/ml actinomycin D for 18 hours to induce apoptosis. Apoptosis was measured by using Cell Death Detection ELISA kit, and the results are represented in Figure 3. As can be seen from Figure 3, it was confirmed that nivocasan prevented TNF-α-induced apoptosis in human chondrocytes in a dose-dependent manner.

### Example 4: Evaluation of efficacy on improving walking ability in animal model of osteoarthritis

Beagle dogs were purchased from Orient Bio, and healthy animals were used for the experiment after a one-week quarantine cycle.

After 1 week of recovery after surgical anterior cruciate ligament transection with medial meniscectomy, forced exercise for 30 minutes every day was carried out to induce osteoarthritis. The surgery for inducing osteoarthritis was performed under anesthesia after the animals were depilated on both knees. After broad disinfection of the incision site with povidone and 70% ethanol, the skin of the right knee was incised. By blunt dissection of the surrounding tissues, the articular surface of the distal end of femur was exposed. An instrument portal was created on the medial articular surface, and the meniscus and anterior cruciate ligament were excised. Wound closure was performed by the use of 4-0 nylon suture. In the case of the left knee, only anterior cruciate ligament transection was performed. In the case of animals in the normal (sham) group, both knees were incised and sutured without excision of meniscus or anterior cruciate ligament. Antibiotics and analgesics were administered for 7 days after surgery. From the 7^{th} day after surgery, forced exercise was carried out for 30 minutes every day until the day before administration of the test substance.

The assessment about walking ability for evaluating the pain status and functional level of osteoarthritis animals was carried out according to the method proposed by Pashuck et al. (Troy D. Pashuck et al., J Orthop Res, 2016, 34(10): 1772-1779), and the lameness score as represented in Table 1 was recorded until the end of the experiment.

**[Table 1]**

| **Score** | **Parameters** |
|---|---|
| 0 | No observable lameness |
| 1 | Intermittent, mild weight bearing lameness with little, if any, change in gait |
| 2 | Moderate weight bearing lameness-obvious lameness with noticeable gait change |
| 3 | Severe weight bearing lameness-"toe-touching" only |
| 4 | Non-weight bearing |

When the lameness score of the osteoarthritis-induced beagle dogs reached an average of 3.5 or higher, the dogs were divided into groups of 5 dogs per group to uniform mean value of each group. The efficacy of nivocasan in an animal model of osteoarthritis was measured by evaluating the lameness score twice a week after a single intra-articular administration. The injection solution for intra-articular administration was prepared by dissolving 0.1, 0.3, 1, 3 or 10 mg of the test substance in 1 mL of 100 mM phosphate buffer solution containing 0.4% (weight ratio) polysorbate 80 (Tween 80) and 0-25 mM NaOH solution, and the final pH was 7.0-7.4. A medium or nivocasan solutions of 0.1, 0.3, 1, 3 and 10 mg/knee doses were administered into the joint cavity of each animal. After anesthetizing the animals, the nivocasan injection solution was administered into the joint cavity of the right knee by the use of a C-arm (Siemens).

The evaluation of the efficacy of nivocasan for improving walking ability by a single intra-articular administration in an animal model of osteoarthritis was conducted twice a week for 3 months, and the results are represented in Figure 4. As can be seen from Figure 4, it was confirmed that nivocasan showed the efficacy of the improvement of walking ability in a dose-dependent manner, and the efficacy was continued up to 3 months.

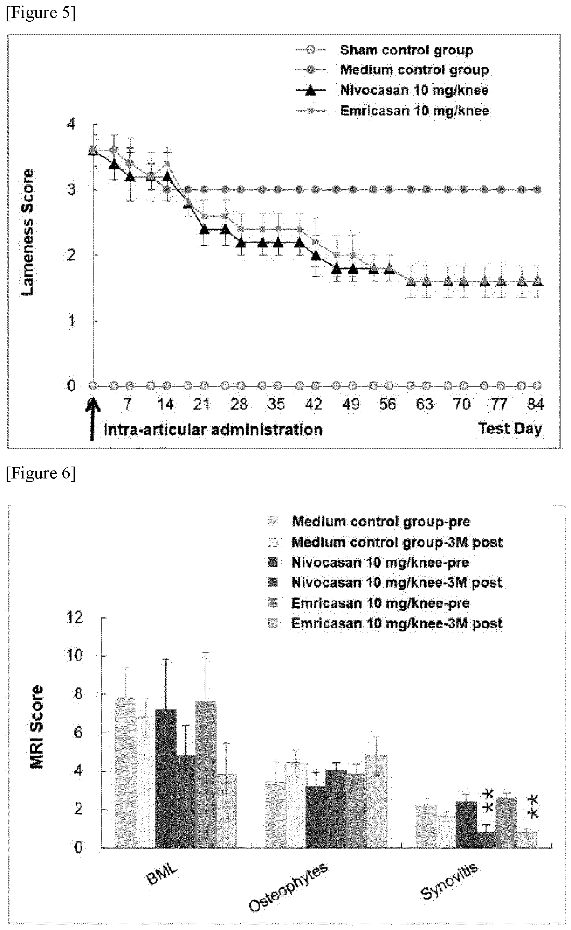

In a dog model of osteoarthritis induced by forced exercise for 30 minutes every day after 1 week of recovery after surgical anterior cruciate ligament transection with medial meniscectomy, as can be seen from Figure 5, a single intra-articular administration of 10 mg of emricasan, a pan-caspase inhibitor, showed the improvement of a walking ability similar to that of 10 mg of nivocasan.

### Example 5: MRI evaluation of effect on joint structure in animal model of osteoarthritis

In a beagle dog model of osteoarthritis induced by daily forced exercise after surgical anterior cruciate ligament transection with medial meniscectomy, the effect on joint structure was evaluated by taking an MRI (SIGNA Pioneer 3.0T MR, GE Healthcare Co., USA) of the joint before a single intra-articular administration and 3 months thereafter. The effect of the test substance on the joint structure was evaluated according to the method suggested by Hunter et al. (David J. Hunter et al., Osteoarthritis and Cartilage 2011, 19, 990-1002) as represented in Table 2. Nivocasan and emricasan, which are pan-caspase inhibitors, showed improvement in bone marrow lesion and synovitis when the MRI was compared before and 3 months after a single intra-articular administration in an animal model of osteoarthritis.

**[Table 2] MRI Score of Joint Structure**

| Parameters | Degrees | Scores |
|---|---|---|
| 1) BML and Cysts | None | 0 |
| | < 33% of subregional volume | 1 |
| | 33 - 66% of subregional volume | 2 |
| | > 66% of subregional volume | 3 |
| 2) Osteophytes | None | 0 |
| | Small | 1 |
| | Moderate | 2 |
| | Marked | 3 |
| 3) Synovitis - effusion | Physiologic amount | 0 |
| | Small - fluid continuous in the retropatellar space | 1 |
| | Medium - fluid continuous in the retropatellar space | 2 |
| | large - evidence of capsular distention | 3 |

### Example 6: Histopathological evaluation of effect on joint in animal model of osteoarthritis

In a beagle dog model of osteoarthritis induced by daily forced exercise after surgical anterior cruciate ligament transection with medial meniscectomy, an autopsy was performed 3 months after a single intra-articular administration, and the joint was collected and fixed. The fixed tissue was subjected to conventional tissue processing such as demineralization, trimming, dehydration, paraffin embedding, sectioning and the like to prepare a specimen for histopathological examination, followed by hematoxylin eosin and safranin-o staining. An optical microscopy (Olympus BX53, Japan) was used to observe histopathological changes.

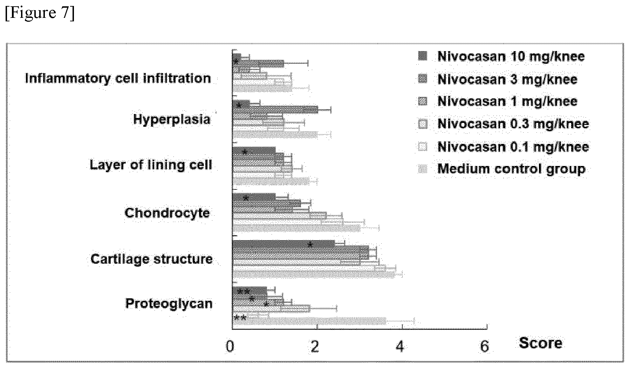

Histopathological evaluation was performed by comparing between groups with scoring as shown in Tables 3 to 8 according to the method proposed by Cook et al. (Cook et al., Osteoarthritis Cartilage. 2010;18 Suppl 3:S66-S79), and the results are represented in Figure 7.

**[Table 3] Grading of Cartilage Structure**

| Severity of pathology characteristics | | Area of section affected | | | |
|---|---|---|---|---|---|
| | | None | Local (Approx 1/3) | Multi-focal (Approx 2/3) | Global (>2/3) |
| A | Normal volume, smooth surface with all zones intact | 0 | 0 | 0 | 0 |
| B | Surface undulations including fissures in surface/upper zone and/or pannus tissue formation on surface | 0 | 1 | 2 | 3 |
| C | Fissures to mid zone and/or erosion of surface/upper zone | 0 | 2 | 4 | 6 |
| D | Fissures that extend to deep zone and/or erosion through mid zone | 0 | 3 | 6 | 9 |
| E | Full thickness loss of cartilage | 0 | 4 | 8 | 12 |

**[Table 4] Grading of Chondrocyte Pathology**

| Severity of pathology characteristics | | Area of section affected | | | |
|---|---|---|---|---|---|
| | | None | Local (Approx 1/3) | Multi-focal (Approx 2/3) | Global (>2/3) |
| A | Normal | 0 | 0 | 0 | 0 |
| B | Loss of cells in the surface zone or relative increased density with occasional superficial clusters | 0 | | 12 | 3 |
| C | Small cell clusters (2-4 cells/cluster) predominate | 0 | 2 | 4 | 6 |
| D | Large cell clusters (≥ 5 cells/cluster) predominate | 0 | 3 | 6 | 9 |
| E | Cell loss (necrosis/apoptosis) predominates | 0 | 4 | 8 | 12 |

**[Table 5] Grading of Proteoglycan Staining**

| Severity of pathology characteristics | | Area of section affected | | | |
|---|---|---|---|---|---|
| | | None | Local (Approx 1/3) | Multi-focal (Approx 2/3) | Global (>2/3) |
| A | Normal | 0 | 0 | 0 | 0 |
| B | Decreased proteoglycan content in surface/upper zone | 0 | 1 | 2 | 3 |
| C | Decreased proteoglycan content into the mid zone | 0 | 2 | 4 | 6 |
| D | Decreased proteoglycan content into the deep zone | 0 | 3 | 6 | 9 |
| E | Full depth decrease in proteoglycan content | 0 | 4 | 8 | 12 |

**[Table 6] Grading of Lining Cells Characteristics**

| Severity of pathology characteristics | | Area of section affected | | | |
|---|---|---|---|---|---|
| | | None | Local (Approx 1/3) | Multi-focal (Approx 2/3) | Global (>2/3) |
| A | 1-2 layers of cells | 0 | 0 | 0 | 0 |
| B | 3-6 layers of cells | 0 | 1 | 2 | 3 |
| C | > 6 layers of cells | 0 | 2 | 4 | 6 |

**[Table 7] Grading of Cell Infiltration Characteristics**

| Severity of pathology characteristics | | Area of section affected | | | |
|---|---|---|---|---|---|
| | | None | Local (Approx 1/3) | Multi-focal (Approx 2/3) | Global (>2/3) |
| A | No cellular infiltration | 0 | 0 | 0 | 0 |
| B | Mild to moderate inflammatory cell infiltrates including small lymphoid follicles | 0 | 1 | 2 | 3 |
| C | Marked, diffuse inflammatory cell infiltrates including large lymphoid follicles | 0 | 2 | 4 | 6 |

**[Table 8] Grading of Hyperplasia Characteristics**

| Severity of pathology characteristics | | Area of section affected | | | |
|---|---|---|---|---|---|
| | | None | Local (Approx 1/3) | Multi-focal (Approx 2/3) | Global (>2/3) |
| A | No villous hyperplasia | 0 | 0 | 0 | 0 |
| B | Short villi | 0 | 1 | 2 | 3 |
| C | Finger-like hyperplasia | 0 | 2 | 4 | 6 |

As can be seen from Figure 7, it was confirmed that dose-related histopathological improvement was shown in the histopathology evaluation of the joints collected by autopsy 3 months after single intra-articular administration of nivocasan.

From the above results of Examples 4 to 6, it was confirmed that nivocasan is not only effective in improving walking ability which reflects the analgesic effect for a long period of at least 3 months or more even with single administration without repeated administration or formulation in sustained release, but is also effective in improving cartilage structure due to the anti-inflammatory effect.

## Claims

1. A pharmaceutical composition for alleviating or treating osteoarthritis having long-lasting effect with a single administration, which comprises a therapeutically effective amount of a caspase inhibitor along with a pharmaceutically acceptable carrier.

2. The pharmaceutical composition for alleviating or treating osteoarthritis according to Claim 1, wherein the caspase inhibitor is selected from the group consisting of nivocasan, IDN-1965, emricasan, MX-1013, pralnacasan, RU-36384, belnacasan and a pharmaceutically acceptable salt thereof.

3. The pharmaceutical composition for alleviating or treating osteoarthritis according to Claim 2, wherein the caspase inhibitor is nivocasan or a pharmaceutically acceptable salt thereof.

4. The pharmaceutical composition for alleviating or treating osteoarthritis according to Claim 2, wherein the pharmaceutically acceptable salt of nivocasan is selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid.

5. The pharmaceutical composition for alleviating or treating osteoarthritis according to Claim 2, wherein the caspase inhibitor is emricasan or a pharmaceutically acceptable salt thereof.

6. The pharmaceutical composition for alleviating or treating osteoarthritis according to Claim 1, wherein the long-lasting effect is analgesic or pain-reducing effect.

7. The pharmaceutical composition for alleviating or treating osteoarthritis according to Claim 1, wherein the long-lasting effect is cartilage structure improvement effect.

8. The pharmaceutical composition for alleviating or treating osteoarthritis according to Claim 1, which is an injection formulation.

9. The pharmaceutical composition for alleviating or treating osteoarthritis according to Claim 8, wherein the injection formulation is for topical administration.

10. The pharmaceutical composition for alleviating or treating osteoarthritis according to Claim 9, wherein the injection formulation is administered into the joint cavity.

11. The pharmaceutical composition for alleviating or treating osteoarthritis according to Claim 1, wherein the administration effect lasts 1 month or more.

12. The pharmaceutical composition for alleviating or treating osteoarthritis according to Claim 11, wherein the administration effect lasts 3 months or more.

13. The pharmaceutical composition for alleviating or treating osteoarthritis according to Claim 12, wherein the administration effect lasts 6 months or more.

14. The pharmaceutical composition for alleviating or treating osteoarthritis according to Claim 1, which is administered with an interval of 4 weeks or more.

15. The pharmaceutical composition for alleviating or treating osteoarthritis according to Claim 14, which is administered with an interval of 8 weeks or more.

16. The pharmaceutical composition for alleviating or treating osteoarthritis according to Claim 15, which is administered with an interval of 12 weeks or more.

17. The pharmaceutical composition for alleviating or treating osteoarthritis according to Claim 16, which is administered with an interval of 24 weeks or more.

18. The pharmaceutical composition for alleviating or treating osteoarthritis according to Claim 1, which is for administration to a mammal.

19. The pharmaceutical composition for alleviating or treating osteoarthritis according to Claim 18, wherein the mammal is a human.

20. The pharmaceutical composition for alleviating or treating osteoarthritis according to Claim 3, wherein the nivocasan is administered into a human in a dose of 1 to 50 mg, based on a free base form.
